# EUROPEAN PATENT APPLICATION

(11) **EP 4 046 645 A1**
(43) Date of publication of application: **24.08.2022**
(21) Application number: 20876177.5
(22) Date of filing: 16.10.2020
(51) Int. Cl.: A61K 35/745, A61P 37/00

(54) **COMPOSITION FOR ENHANCING OR IMPROVING IMMUNE SYSTEM COMPRISING BIFIDOBACTERIUM BIFIDUM**

(30) Priority: 18.10.2019 KR 20190130275
(71) Applicant: Genome and Company, Seongnam-si, Gyeonggi-do 13486 (KR); Gwangju Institute of Science and Technology, Gwangju 61005 (KR)
(72) Inventor: PARK, Hansoo, Seongnam-si Gyeonggi-do 13486 (KR); SOHN, Jinyoung, Seongnam-si Gyeonggi-do 13486 (KR); KIM, Yul Ha, Seongnam-si Gyeonggi-do 13486 (KR)
(74) Representative: Rückerl, Florian
(86) International application number: PCT/KR2020/014203
(87) International publication number: WO 2021/075928

(57) **Abstract**

The present invention relates to a composition for enhancing or improving immunity comprising a Bifidobacterium bifidum. In addition, it can be used to prevent, treat, or improve immune diseases caused by immunodeficiency, immunosuppression, or immune system damage. It can be provided as a food composition or a pharmaceutical composition, and the food composition can be provided in various forms such as probiotic products, health functional supplements, animal feed, and the like.

## Description

### [Technical Field]

The present invention relates to a composition for enhancing or improving immune system comprising a Bifidobacterium bifidum. The composition according to the present invention may be provided as a food composition or a pharmaceutical composition.

### [Background Art]

The World Health Organization (WHO) and the Food and Agriculture Organization (FAO) have defined probiotics as "live microorganisms which when administered in adequate amounts confer a health benefit on the host", and the Korea Food & Drug Administration (KFDA) defined it as "live microorganisms that have a beneficial effect on health". Examples typically used as such probiotics include Lactobacillus, Bifidobacterium, etc.

The Bifidobacterium is a Gram-positive anaerobic bacterium, which exists in the gastrointestinal tract, vagina, or mouth of mammals including humans, and is one of the major bacteria constituting the gastrointestinal microbiota of mammals. Species belonging to the genus Bifidobacterium include, for example, B. actinocoloniiforme, B. adolescentis, B. angulatum, B. animalis, B. aquikefiri, B. asteroides, B. biavatii, B. bifidum, B. bohemicum, B. bombi, B. boum, B. breve, B. callitrichos, B. catenulatum, B. choerinum, B. commune, and B. coryneforme. In addition, various subspecies and strains exist in large numbers for each species, and new subspecies and strains are still being discovered.

These bacteria have very different characteristics from species to species. In addition, even in the same species, an effect and its level are different for each subspecies and strain.

For example, Korean Patent No. 10-1970148 discloses a novel Lactobacillus strain with excellent acid resistance and bile resistance, and shows in experiments that the level of intestinal adhesion ability is different for each strain belonging to the Lactobacillus. As another example, Korean Patent No. 10-1589465 discloses a novel Bifidobacterium breve strain for growth promotion, and shows in experiments that the level of characteristics such as intestinal adhesion ability is different for each strain strains belonging to the Bifidobacterium breve species.

In addition, there has been recently an increasing interest in immunity. In particular, because of environmental pollution, lack of exercise, dietary changes, etc., there is a continuous demand for substances that can exhibit immunomodulatory effects. A lactic acid bacterium is one of such substances, and many studies have conducted.

### [Technical Problem]

The inventors have conducted various studies on novel substances that can exhibit a significant immunity enhancing or improving effect. As a result, it has been experimentally proven that Bifidobacterium bifidum, particularly Bifidobacterium bifidum MG731, shows a remarkably excellent immune enhancing effect, and completed the present invention.

### [Technical Solution]

One aspect of the present invention provides a composition for enhancing or improving immunity, comprising Bifidobacterium bifidum.

Preferably, the Bifidobacterium bifidum is a Bifidobacterium bifidum MG731.

In the present invention, "Bifidobacterium bifidum" may be used interchangeably with "B. bifidum".

As used herein, the term "Bifidobacterium bifidum MG731" refers to a Bifidobacterium bifidum MG731 strain. The strain is interpreted to include a strain itself, a culture of the strain, a lysate of the strain, an extract of the strain, and a cytoplasmic fraction obtained by lysing the strain. In addition, the strain is interpreted to include both live cells and dead cells.

The MG731 is a Bifidobacterium bifidum strain deposited by Mediogen at the Korean Collection for Type Cultures (KCTC) of Korea Research Institute of Bioscience and Biotechnology (KRIBB) on January 4, 2018 with accession number KCTC13452BP, and the nucleotide sequence of its 16S rRNAgene is represented by SEQ ID NO. 1.

When the composition according to the present invention is administered to a subject in need of enhancing or improving immunity, an expression or activity of one or more of IL-7, IL-15, and INF-γ can be increased, and an expression or activity of TGF-β can be decreased. Therefore, the composition according to the present invention can exhibit an excellent immune enhancing effect, and, for example, can be used for a subject in need of prevention, treatment or improvement of immune diseases.

As used herein, the term "immune disease" refers to a disease caused by immunodeficiency, immunosuppression, or damage to the immune system, and for example, may be, but not limited to, an infectious disease or an inflammatory disease.

As used herein, the term "subject" includes a human or any non-human animal. The non-human animal may be, for example, a vertebrate, such as a primate, dog, cow, horse, pig, chicken, duck, goose, rodent such as mouse, rat, guinea pig, and the like. The term "subject" may be used interchangeably herein with "individual" or "patient".

As used herein, the term "immunity" is used interchangeably with "immune system", "immune function" or "immune activity".

According to another aspect of the present invention, the composition of the present invention may be a food composition.

The food may be a health functional food or a dietary supplement. As used herein, the term "health functional food" refers to a food manufactured and processed in the form of tablets, capsules, powders, granules, liquids, pills, etc. using raw materials or ingredients useful to the human body. Herein, "functional" or "functionality" refers to obtaining effects useful for health purposes such as regulating nutrients or physiological actions with respect to the structure and function of the human body.

The effective amount of Bifidobacterium bifidum MG731 comprised in the food composition according to the present invention may be appropriately determined depending on the purpose of use such as prevention, improvement, or therapeutic treatment. For example, Bifidobacterium bifidum MG731 may be comprised in an amount of 0.001 to 20 percent by weight (wt%), 0.001 to 15 wt%, or 0.001 to 10 wt%, in the food composition. In the case of health drinks, it may be comprised, for example in an amount of 0.01 to 2 g, or 0.02 to 2 g based on 100 mL. However, in the case of long-term ingestion for the purpose of health control, it is possible to ingest in an amount less than the above range. The Bifidobacterium bifidum MG731 added to the food composition in the process of preparing it may be appropriately increased or decreased as necessary.

The food composition may be in any one formulation, such as pills, tablets, granules, powders, capsules, liquids, and solutions, but is not limited thereto.

The type of the food is not limited. For example, the food composition according to the present invention can be commercialized or formulated as yogurt, dairy products, meat, sausage, bread, chocolate, candy, snacks, confectionery, pizza, noodles, gum, ice cream, soup, beverage, tea, drinks, alcoholic beverages, vitamin complexes, etc., but is not limited thereto. It may include all food products.

The food composition of the present invention may contain various sweeteners or natural carbohydrates as additional ingredients like conventional food, and there is no limitation on the type if it does not inhibit or offset the effect of the active ingredient according to the present invention. The natural carbohydrates are, for example, monosaccharides such as glucose and fructose, disaccharides such as maltose and sucrose, polysaccharides such as dextrin and cyclodextrin, and sugar alcohols such as xylitol, sorbitol, and erythritol. The sweetener may be, for example, a natural sweetener such as thaumatin or stevia extract, or a synthetic sweetener such as saccharin or aspartame.

When the food composition of the present invention is a beverage composition, there is no limitation on the liquid component if it contains Bifidobacterium bifidum MG731 as an active ingredient. Like a conventional beverage, it may contain various flavoring agents or natural carbohydrates as additional ingredients.

When the food composition of the present invention is an animal feed composition, there is no limitation on animal feed if it contains Bifidobacterium bifidum MG731 as an active ingredient. The animal feed may be provided in the form of foods, snacks or dietary supplements to enhance or improve immunity of animals, or to prevent, treat or improve diseases caused by immunodeficiency, immunosuppression, or immune system damage. Said term "animal" refers to any non-human animal. The non-human animal may be, for example, vertebrates such as primates, dog, cow, horse, pig, chicken, duck, goose, rodent, such as mouse, rat, guinea pig, and the like.

According to another aspect of the present invention, the composition of the present invention may be a pharmaceutical composition.

In the pharmaceutical composition according to the present invention, each term has the same meaning as described above in the food composition unless otherwise specified.

The pharmaceutical composition may be administered in an effective amount to a subject in need thereof in order to enhance or improve his/her immunity, or to prevent, treat or improve diseases or symptoms caused by immunodeficiency, immunosuppression, or immune system damage.

As used herein, the term "treatment" or "treating" refers to any action in which immune system is enhanced or improved, or immune-related diseases are improved or cured by administration of the composition according to the present invention. In addition, the term "prevention" or "preventing" as used herein refers to any action that suppresses or delays a decrease in immunity, an occurrence of immune-related diseases, or a development of symptoms thereof by administration of the composition according to the present invention. In addition, the term "improvement" or "improving" as used herein refers to any action that enhances or improves the immune system, or maintains the level of immunity or prevents weakening of immunity, or at least reduces, improves, or benefits the symptoms of immune-related diseases by administration of the composition according to the present invention.

The effective amount may be a "therapeutically effective amount" or a "prophylactically effective amount". As used herein, the term "therapeutically effective amount" refers to, when a drug or a therapeutic agent is used alone or in combination with other therapeutic agents, any amount capable of having a decrease in the severity of an immune-related disease, an increase in the frequency and duration of the immune-related disease symptom-free period, or a prevention of damage or impairment due to suffering from an immune-related disease. In addition, as used herein, the term "prophylactically effective amount" refers to any amount capable of preventing, suppressing or delaying the risk of suffering from reduced immunity, or occurrence or recurrence of immune-related diseases. The level of effective amount may be determined by a person skilled in the art such as a doctor depending on factors including severity, age, sex, drug activity, drug sensitivity, administration time, administration route and excretion rate, treatment period, concurrent drugs, and other factors well known in the medical field.

As used herein, the term "administration" or "administering" refers to the physical introduction of the composition to a subject using any various methods and delivery systems known to a person skilled in the art. The Route of administration for the pharmaceutical composition of the present invention includes, for example, oral routes, or routes of administration by intravenous, intramuscular, subcutaneous, intraperitoneal, spinal or other parenteral routes, such as by injection or infusion, but is not limited thereto.

The administrations of the composition of the present invention may be, for example, performed once, multiple times, or over one or more extended periods of time. For example, it may be administered in an amount of 0.1 to 100 mg/kg once to several times a day, or administered at intervals of several days to several months. In addition, the administration frequency may be increased or decreased depending on the administration route, disease severity, sex, weight, age, dosage, and the like.

The pharmaceutical composition of the present invention may further comprise suitable carriers, excipients or diluents commonly used in its preparation. The carriers, excipients or diluents that may be contained in the composition include, for example, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil, but are not limited thereto.

The composition such as a food composition or a pharmaceutical composition may be administered in combination with other substances or ingredients. In this case, the composition of the present invention and other substances or ingredients may be administered sequentially, simultaneously, or separately. The other substances or ingredients may be included without limitation, provided that they do not inhibit or offset the efficacy of Bifidobacterium bifidum MG731.

The composition comprising Bifidobacterium bifidum MG731 according to the present invention may be formulated to be administered simultaneously, separately or sequentially with other substances or components. For example, Bifidobacterium bifidum MG731 and an immune enhancing component may be administered simultaneously as one formulation, or may be administered simultaneously or sequentially as separate formulations.

For simultaneous, separate or sequential administration, Bifidobacterium bifidum MG731 contained in the composition of the present invention and other substances or components may be formulated separately in each container, or may be formulated together in the same container. In addition, the effective amount, administration time, administration interval, administration route, etc. of the Bifidobacterium bifidum MG731 contained in the composition of the present invention and other substances or components may be the same or different from each other.

According to another aspect of the present invention, the present invention provides a method for enhancing or improving immunity of a subject, comprising administering a Bifidobacterium bifidum to the subject in need of enhancing or improving immunity.

According to another aspect of the present invention, it provides a method for preventing, treating or improving a disease caused by an immunodeficiency, immunosuppression, or immune system damage of subject, comprising administering to the subject a Bifidobacterium bifidum. The subject may be, for example, suffering from an immune disease or having a high risk of an immune disease.

In the method according to the present invention, each term has the same meaning as described above with respect to the composition unless otherwise specified.

Preferably, the Bifidobacterium bifidum is Bifidobacterium bifidum MG731.

In the method according to the present invention, the Bifidobacterium bifidum may be administered to a subject simultaneously, sequentially, or separately with other immunotherapy or immune enhancing agents. The "simultaneous" administration means that Bifidobacterium bifidum MG731 and another substance are administered at once in one formulation, or Bifidobacterium bifidum MG731 and another substance are administered at one time in separate formulations, in which the route of administration thereof may be different each other. The "sequential" administration refers to a relatively continuous administration of the Bifidobacterium bifidum MG731 and other substances, allowing the administration interval to a minimum. The "separate" administration refers to an administration of the Bifidobacterium bifidum MG731 and other substances at predetermined time intervals. The administration method of the Bifidobacterium bifidum MG731 and other substances may be appropriately selected by a doctor or expert in the art in consideration of the treatment efficacy or side effects of subject.

The composition according to the present invention can increase an expression or activity of one or more of IL-7, IL-15 and INF-γ, and decrease an expression or activity of TGF-β. Accordingly, it can exhibit a significantly excellent immune enhancing effect.

Accordingly, it can be provided as a food composition or a pharmaceutical composition, and the food composition can be provided in various forms such as probiotic products, health functional supplements, animal feed, and the like.

In addition, it can be used to prevent, treat, or improve immune diseases caused by immunodeficiency, immunosuppression, or immune system damage.

### [Brief Description of Figures]

FIG. 1 is a graph showing the relative expression levels of IL-7, IL-15, IFN-γ and TGF-β in THP-1 cells treated with or not treated with Bifidobacterium bifidum MG731, respectively.
FIG. 2 is a graph showing the relative expression levels of IL-7, IL-15, IFN-γ and TGF-β in Jurkat cells treated with or not treated with Bifidobacterium bifidum MG731, respectively.
FIG. 3 is a graph showing the relative expression levels of IL-7, IL-15, IFN-γ and TGF-β in human monocyte cells treated with or not treated with Bifidobacterium bifidum MG731, respectively.

### [Examples]

Hereinafter, the present invention will be described in more detail by Examples. However, these examples are only for illustrative purposes, and the scope of the present invention is not limited thereto.

### Example 1. Preparation of Bifidobacterium bifidum

MG731 strains (Accession No. KCTC13452BP) were subcultured with two passages in BL broth. Then, it was incubated in RPMI 90% BL 10% medium for 24 hours. After collecting the MG731 strain and measuring the absorbance at O.D 600, a strain diluent was prepared so as to have a final concentration of 1×10⁷ CFU/ml.

### Example 2. Evaluation of Immunomodulatory Efficacy

MG731 was cultured in BL broth under anaerobic conditions at 37°C. Human blood used in the test was put into a 10 mL tube coated with EDTA (or heparin) and mixed with PBS at a ratio of 1:1. Then, Ficoll-Paque PLUS was placed in a 50 mL tube, and the blood sample was added. After centrifugation, PBMCs (peripheral blood mononuclear cells) were harvested.

Human monocytes were isolated from the PBMCs collected above using a human monocyte isolation kit. The Jurkat cell line, which is a human T cell, was cultured in a 75T flask at 37°C and 5% CO₂ condition using RPMI 1640 medium containing 10% fetal bovine serum (FBS). In addition, the THP-1 cell line, which is a human monocyte cell, was cultured in a 75T flask at 37°C and 5% CO₂ condition using RPMI 1640 medium containing 10% FBS and 0.1% 2-mercaptoethanol.

THP-1 cells, Jurkat cells, and human monocyte cells were counted, respectively, and then diluted in a cell culture without antibiotics and seeded in lower wells of the transwell to be 1×10⁶ cells/well/0.8mL. MG731 was diluted in the same cell culture and seeded in insert wells to be 1×10⁷ CFU/well/0.2mL.

After culturing for 24 hours, the insert well containing the Bifidobacterium bifidum MG731 strain was removed. Then, RNA of cells in each well was extracted with Trizol, and cDNA was synthesized.

After that, the expression levels of IL-7, IL-15, IFN-γ and TGF-β were measured through real-time PCR using the cDNA.

IL-7 and IL-15 are known as factors inducing proliferation, differentiation, and survival of T cells in early immune response. In addition, IFN-γ is an immune factor secreted by cytotoxic T cells and is known as a representative cytokine to enhance an immune function. On the other hand, TGF-β is known as one of the factors having an immunosuppressive function. Accordingly, increasing the expression of IL-7, IL-15 and IFN-γ and/or inhibiting the expression of TGF-β indicates that there is an immune enhancing effect.

Primer sequences for real-time PCR are shown in Table 1 below.

**Table 1**

| Primer | Sequence | |
|---|---|---|
| IL-7 | F | CTCCCCTGATCCTTGTTCTG |
| | R | TCATTATTCAGGCAATTGCTACC |
| IL-15 | F | CCAACTGGGTGAATGTAATAAGTGA |
| | R | TGCAACTGGGGTGAACATCA |
| IFN-γ | F | GTCCAACGCAAAGCAATACA |
| | R | CTCTTCGACCTCGAAACAGC |
| TGF-β | F | GGGACTATCCACCTGCAAGA |
| | R | CCTCCTTGGCGTAGTAGTCG |

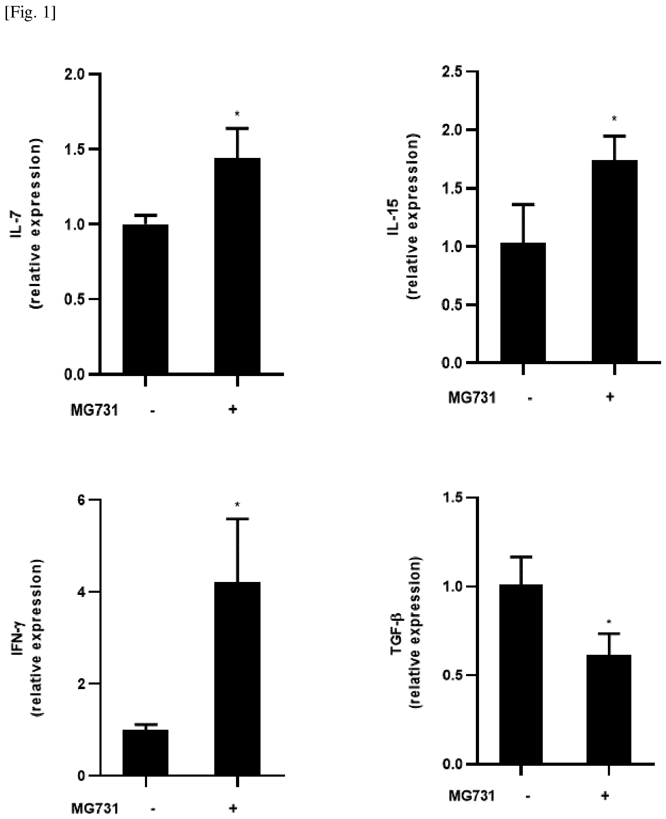

As seen in FIG. 1 showing the relative expression levels of IL-7, IL-15, IFN-γ and TGF-β in THP-1 cells treated with or not treated with Bifidobacterium bifidum MG731, the expression levels of IL-7, IL-15 and INF-γ were increased by 1.44-fold, 1.68-fold, and 4.19-fold, respectively, compared to that of the control group. On the contrary, the expression level of TGF-β was decreased by 0.88-fold.

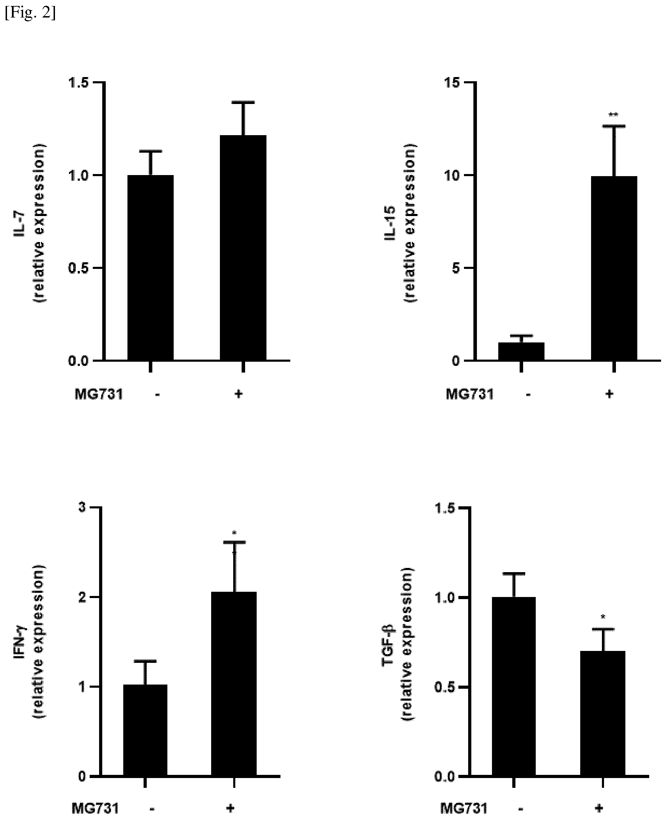

In addition, as seen in FIG. 2 showing the relative expression levels of IL-7, IL-15, IFN-γ and TGF-β in Jurkat cells treated with or not treated with Bifidobacterium bifidum MG731, the expression levels of IL-15 and INF-γ were increased by 9.61-fold and 2.01-fold, respectively, compared to that of the control group. On the contrary, the expression level of TGF-β was decreased by 0.7-fold.

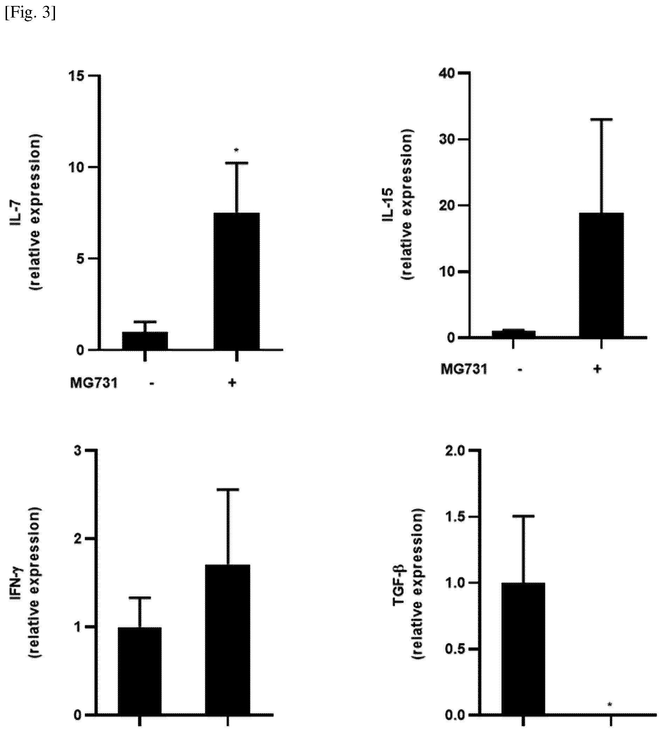

In addition, as seen in FIG. 3 showing the relative expression levels of IL-7, IL-15, IFN-γ and TGF-β in human monocyte cells treated with or not treated with Bifidobacterium bifidum MG731, the expression level of IL-7 was increased by 7.55-fold compared to that of the control group, whereas the expression level of TGF-β hardly appeared.

This means that in all of THP-1, Jurkat and human monocyte cells which are human immune cells, the expression of IL-7 and IL-15 is increased by Bifidobacterium bifidum MG731, thereby activating the proliferation and differentiation of T cells, and the expression of IFN-γ is increased while the expression of TGF-β is decreased, thereby enhancing or improving the immune function.

## Claims

1. A composition for enhancing or improving immunity comprising a Bifidobacterium bifidum MG731.

2. The composition for enhancing or improving immunity of claim 1, wherein the Bifidobacterium bifidum MG731 is a strain, a culture of the strain, a lysate of the strain or an extract of the strain.

3. The composition for enhancing or improving immunity of claim 1, wherein the composition increases an expression or activity of one or more of IL-7, IL-15 and IFN-γ.

4. The composition for enhancing or improving immunity of claim 1, wherein the composition inhibits an expression or activity of TGF-β.

5. The composition for enhancing or improving immunity of claim 1, wherein the composition is a food composition.

6. The composition for enhancing or improving immunity of claim 5, wherein the composition is for improving or preventing immune diseases.

7. The composition for enhancing or improving immunity of claim 1, wherein the composition is a pharmaceutical composition.

8. The composition for enhancing or improving immunity of claim 7, wherein the composition is for treating or preventing immune diseases.


<110>     Genome and Company
          Gwangju Institute of Science and Technology

<120>     COMPOSITION FOR ENHANCING OR IMPROVING IMMUNE ACTIVITY COMPRISING
          BIFIDOBACTERIUM BIFIDUM

<130>     PCT20022

<150>     KR 10-2019-0130275
<151>     2019-10-18

<160>     1

<170>     KoPatentIn 3.0

<210>     1
<211>     1447
<212>     RNA
<213>     Bifidobacterium bifidum

<220>
<221>     rRNA
<222>     (1)..(1447)
<223>     MG731 16S rRNA


<400>     1
agacgctggc ggcgtgctta acacatgcaa gtcgaacggg atccatcggg ctttgcttgg        60

tggtgagagt ggcgaacggg tgagtaatgc gtgaccgacc tgccccatgc tccggaatag       120

ctcctggaaa cgggtggtaa tgccggatgt tccacatgat cgcatgtgat tgtgggaaag       180

attctatcgg cgtgggatgg ggtcgcgtcc tatcagcttg ttggtgaggt aacggctcac       240

caaggcttcg acgggtagcc ggcctgagag ggcgaccggc cacattggga ctgagatacg       300

gcccagactc ctacgggagg cagcagtggg gaatattgca caatgggcgc aagcctgatg       360

cagcgacgcc gcgtgaggga tggaggcctt cgggttgtaa acctcttttg tttgggagca       420

agccttcggg tgagtgtacc tttcgaataa gcgccggcta actacgtgcc agcagccgcg       480

gtaatacgta gggcgcaagc gttatccgga tttattgggc gtaaagggct cgtaggcggc       540

tcgtcgcgtc cggtgtgaaa gtccatcgct taacggtgga tctgcgccgg gtacgggcgg       600

gctggagtgc ggtaggggag actggaattc ccggtgtaac ggtggaatgt gtagatatcg       660

ggaagaacac cgatggcgaa ggcaggtctc tgggccgtca ctgacgctga ggagcgaaag       720

cgtggggagc gaacaggatt agataccctg gtagtccacg ccgtaaacgg tggacgctgg       780

atgtggggca cgttccacgt gttccgtgtc ggagctaacg cgttaagcgt cccgcctggg       840

gagtacggcc gcaaggctaa aactcaaaga aattgacggg ggcccgcaca agcggcggag       900

catgcggatt aattcgatgc aacgcgaaga accttacctg ggcttgacat gttcccgacg       960

acgccagaga tggcgtttcc cttcggggcg ggttcacagg tggtgcatgg tcgtcgtcag      1020

ctcgtgtcgt gagatgttgg gttaagtccc gcaacgagcg caaccctcgc cccgtgttgc      1080

cagcacgtta tggtgggaac tcacggggga ccgccggggt taactcggag gaaggtgggg 1140

atgacgtcag atcatcatgc cccttacgtc cagggcttca cgcatgctac aatggccggt 1200

acagcgggat gcgacatggc gacatggagc ggatccctga aaaccggtct cagttcggat 1260

cggagcctgc aacccggctc cgtgaaggcg gagtcgctag taatcgcgga tcagcaacgc 1320

cgcggtgaat gcgttcccgg gccttgtaca caccgcccgt caagtcatga aagtgggcag 1380

cacccgaagc cggtggccta accccttgtg ggatggagcc gtctaaggtg aggctcgtga 1440

ttgggac 1447